(19) Europäisches Patentamt
European Patent Office
Office européen des brevets

(11) Veröffentlichungsnummer : **0 046 194**
**B1**

(12) **EUROPÄISCHE PATENTSCHRIFT**

(45) Veröffentlichungstag der Patentschrift : **05.10.83**

(51) Int. Cl.³ : **C 07 C 63/04**, C 07 C 63/70, C 07 C 51/29

(21) Anmeldenummer : **81105451.9**

(22) Anmeldetag : **13.07.81**

(54) **Verfahren zur Herstellung von o,o-disubstituierten Benzoesäuren und ihren Salzen.**

(30) Priorität : **20.08.80 DE 3031364**

(43) Veröffentlichungstag der Anmeldung : **24.02.82 Patentblatt 82/08**

(45) Bekanntmachung des Hinweises auf die Patenterteilung : **05.10.83 Patentblatt 83/40**

(84) Benannte Vertragsstaaten : **BE CH DE FR GB IT LI NL**

(56) Entgegenhaltungen :
**DE B 1 171 900**
**GB A 497 353**

**Houben-Weyl, Methoden der Organischen Chemie, Vol. VIII, Stuttgart (1952) S. 415**

**Ullmanns Encyklopädie der technischen Chemie, 4. Aufl., Band 10, Weinheim (1975) S. 455-462**

(73) Patentinhaber : **BASF Aktiengesellschaft**
**Carl-Bosch-Strasse 38**
**D-6700 Ludwigshafen (DE)**

(72) Erfinder : **Ruge, Bernd, Dr.**
**Bruesseler Ring 43**
**D-6700 Ludwigshafen (DE)**

**EP 0 046 194 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von o,o-disubstituierten Benzoesäuren und ihren Salzen

Die Erfindung betrifft ein Verfahren zur Herstellung von o,o-disubstituierten Benzoesäuren und ihren Salzen durch Umsetzungen von aromatischen Ketonen mit Hypohalogeniten und basischen Verbindungen in Gegenwart eines Phasentransferkatalysators und gewünschtenfalls Umsetzung des gebildeten Salzes mit Säure.

Es ist bekannt, daß Arylmethylketone mit Hilfe von Hypohalogeniten in Gegenwart von Basen zu Aryl-α,α,α-trihalogenmethylketonen umgesetzt und diese wiederum mit den Basen zu den entsprechenden Carbonsäuren hydrolysiert werden (Houben-Weyl, Methoden der Organischen Chemie, Band VIII, Seite 415 (4. Auflage)). Die Anwendung dieser Methode stößt jedoch im Falle von o-disubstituierten, aromatischen Ketonen auf erhebliche Schwierigkeiten. So erfordert die Umsetzung von o,o-disubstituierten Arylketonen einen hohen Überschuß an Hypohalogenit sowie Reaktionszeiten von mehreren Tagen. Zum Beispiel ergibt die Umsetzung von 2,4,6-Trimethylacetophenon nur mit einem sechsfachen stöchiometrischen Überschuß an Natriumhypochlorit bei einer Reaktionsdauer von 3 Tagen α,α,α-Trichlor-2,4,6-trimethylacetophenon in 88 % Ausbeute (JACS *52,* 3269-3275 (1930)). α,α,α-Trichlor-2,4,6-trimethylacetophenon widersteht der Zersetzung durch Alkalien in unerwarteter Weise. Erst zweistündiges Kochen mit 40-gewichtsprozentiger Natronlauge ergibt Trimethylbenzoesäure (Chem. Ber. *63,* 2455-2463 (1930)). In ähnlicher Weise werden für die Umsetzung von 2,4,6-Trichloracetophenon zu α,α,α-2,4,6-Hexachloracetophenon ein 11,5-facher stöchiometrischer Überschuß an Natriumhypochlorit und eine Reaktionsdauer von 4 Tagen für eine Ausbeute von 21 % benötigt. 24-stündiges Kochen mit 20-gewichtsprozentiger, wäßriger Natronlauge ergibt lediglich geringe Mengen 2,4,6-Trichlorbenzoesäure (JACS *59,* 1508-1510 (1937)).

Die deutsche Auslegeschrift 17 68 050 lehrt die Verwendung quaternärer Verbindungen, auch bei der Reduktion von Carbonylverbindungen zu Alkoholen. Die 18 Ausführungsbeispiele zeigen zwar zahlreiche Beispiele für die Herstellung von Cyaniden, Halogeniden und organischen Sulfonaten. In keinem Beispiel wird ein aromatisches Keton gezeigt, auf der anderen Seite wird die Verwendung von ungesättigten Aliphaten hervorgehoben. Die einzige Herstellungsmethode von Benzoesäure verwendet Stilben und Kaliumpermanganat als Komponenten. Die Ausbeute von 53 % (Beispiel 14) ist unbefriedigend.

Weitere bekannte Methoden, beispielsweise die Herstellung von 2,4,6-Trimethylbenzoesäure, Umsetzung von Mesitylmagnesiumbromid mit Kohlendioxid (Org. Synth. Coll. Vol. 3, Seite 553) oder Friedel-Crafts-Acylierung von Mesitylen mit Oxalylchlorid (Org. Synth., Vol. 44, Seiten 69-71 (1964)) sind entweder technisch kaum realisierbar oder wegen der hohen Kosten der Ausgangsstoffe wirtschaftlich unbefriedigend.

Es wurde nun gefunden, daß man o,o-disubstituierte Benzoesäuren der Formel

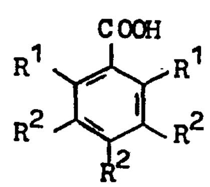

(I)

worin die einzelnen Reste $R^1$ und $R^2$ gleich oder verschieden sein können und jeweils ein Halogenatom, einen aliphatischen, cycloaliphatischen, araliphatischen oder aromatischen Rest oder eine Alkoxygruppe bedeuten, die einzelnen Reste $R^2$ auch jeweils für ein Wasserstoffatom stehen können, oder ihren Salzen, durch Umsetzung von Ketonen mit Hypohalogeniten und basischen Verbindungen und gewünschtenfalls Umsetzung des gebildeten Salzes mit Säure vorteilhaft erhält, wenn man o,o-disubstituierte aromatische Ketone der Formel

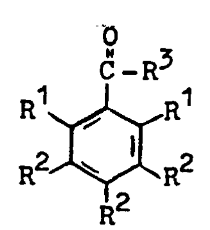

(II)

worin $R^1$ und $R^2$ die vorgenannte Bedeutung besitzen und $R^3$ einen aliphatischen Rest bezeichnet, in Gegenwart von Phasentransferkatalysatoren der Formel

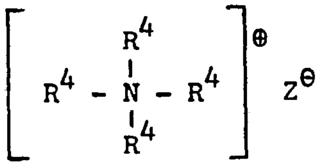

(III)

worin die einzelnen Reste $R^4$ gleich oder verschieden sein können und jeweils einen Alkylrest mit 1 bis 18 Kohlenstoffatomen oder einen Aralkylrest mit 7 bis 12 Kohlenstoffatomen bedeuten, Z für ein Säureanion steht, umsetzt.

Die Umsetzung kann für den Fall der Verwendung von 2,4,6-Trimethylacetophenon und Natriumhypochlorit-Lösung durch die folgenden Formeln wiedergegeben werden :

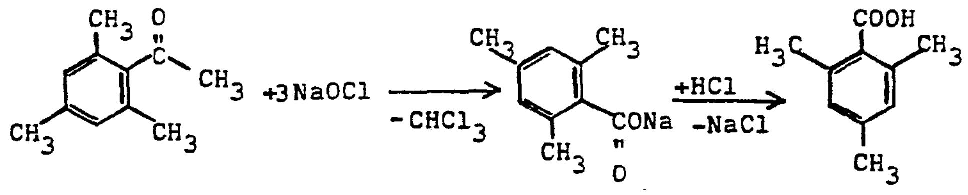


Im Vergleich zu den bekannten Verfahren liefert das Verfahren nach der Erfindung überraschend auf einfacherem und wirtschaftlicherem Wege eine große Zahl von o,o-disubstituierten Benzoesäuren in besserer Ausbeute, Raum-Zeit-Ausbeute und Reinheit, gerade auch im großtechnischen Maßstab und im kontinuierlichen Betrieb. Diese vorteilhaften Ergebnisse sind im Hinblick auf den Stand der Technik, wo o,o-disubstituierte Benzoesäuren nur auf unbefriedigendem Wege hergestellt werden können, überraschend.

Im Hinblick auf J. Chem. Ed., Vol. 55 (1978), Seite 431, rechte Spalte, hätte man als Ergebnis der Oxidation Arenoxide bzw. Benzaldehydverbindungen erwarten müssen. Berücksichtigt man die deutsche Offenlegungsschrift 17 68 050, Beispiel 14, ist es überraschend, daß organische Lösungsmittel eingespart werden und trotzdem bessere Ausbeuten erzielt werden. Auch konnte aufgrund vorgenannter Arbeit in J. Chem. Ed. (loc. cit.), insbesondere Seite 431, nicht erwartet werden, daß sich Carbonsäuren bilden, sondern man mußte mit heterogenen Mischungen und der Bildung von Substituierten Benzaldehyden rechnen. Wie Seite 231 der vorgenannten Publikation ausdrücklich feststellt, oxidiert Hypochlorit Benzylalkohol zu den entsprechenden Benzaldehyden ohne Überoxidation. Auch ist es überraschend, daß trotz der sterischen Hinderung infolge der o,o-Substituenten die Reaktion nicht zu chlorierten Zwischenprodukten sondern zu den Benzoesäuren I führt.

Bevorzugte Ausgangsstoffe II und dementsprechend bevorzugte Endstoffe der Formel I sind solche, in deren Formeln die einzelnen Reste $R^1$ und $R^2$ gleich oder verschieden sein können und jeweils eine Alkoxygruppe oder einen Alkylrest mit jeweils 1 bis 18, insbesondere 1 bis 7 Kohlenstoffatomen, einen Cycloalkylrest mit 5 bis 8 Kohlenstoffatomen, einen Aralkylrest oder Alkylarylrest mit jeweils 7 bis 12 Kohlenstoffatomen oder einen Phenylrest, ein Jodatom, Bromatom oder insbesondere ein Chloratom bedeuten, die Reste $R^2$ auch jeweils ein Wasserstoffatom bezeichnen können, $R^3$ für einen Alkylrest mit jeweils 1 bis 18, insbesondere 1 bis 7 Kohlenstoffatomen steht. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Es kommen z. B. als Ausgangsstoffe II in Frage : Am aromatischen Kern in 2- und 6-Stellung zweifach oder in 2-, 4- und 6-Stellung bzw. 2-, 5-, 6-Stellung dreifach oder in 2-, 4- und 6-Stellung bzw. 2-, 5-, 6-Stellung dreifach oder in 2-, 4-, 5-, 6-Stellung vierfach gleich oder verschieden durch die Methyl-, Äthyl-, Propyl-, Isopropyl-, Butyl-, Isobutyl-, sek.-Butyl-, tert.-Butyl-, Methoxy-, Äthoxy-, Propoxy-, Isopropoxy-, Butoxy-, Isobutoxy-, sek.-Butoxy-, tert.-Butoxy-, Cyclohexyl-, Cyclopentyl-, Benzyl-, Phenyl-gruppe und/oder Chlor oder Brom substituiertes Acetophenon ; homologe Äthyl-, Propyl-, Isopropyl-, Butyl-, Isobutyl-, sek.-Butyl-, Pentyl-, Hexyl-, Heptyl-, Octyl-phenylketone.

Als Oxidationsmittel verwendet man Hypohalogenite, in der Regel Hypochlorite und Hypobromite in wäßrigem Medium, in der Regel in Gestalt von entsprechenden wäßrigen, alkalischen Lösungen. Vorteilhaft gelangen wäßrige Suspensionen von 1 bis 10 Gewichtsprozent Ausgangsstoff II zur Anwendung. Die wäßrigen Hypohalogenitlösungen, vorzugsweise Hypochloritlösungen enthalten im allgemeinen von 5 bis 15, vorzugsweise von 12 bis 14 Gewichtsprozent Hypohalogenit, vorzugsweise Hypochlorit, und können zweckmäßig zusätzlich 0,2 bis 1 Mol Alkalihydroxid je Mol Hypohalogenit, vorzugsweise Hypochlorit, enthalten. Bei dem Überschuß von mehr als 0,2 Mol Alkalihydroxid wird das gegebenenfalls im Katalysator oder Hypochlorit enthaltene oder durch den Katalysator gebundene Alkali nicht eingerechnet. Im Ausgangsgemisch beider Ausgangsstoffe kommen im allgemeinen Mengen von insgesamt 3 bis 6, vorzugsweise 4 bis 5 Mol Hypochlorit und zweckmäßig insgesamt 0,2 bis 6 Mol, vorzugsweise 1 bis 4 Mol Alkalihydroxid (nicht eingerechnet das im Hypohalogenit enthaltene Alkali), bezogen auf 1 Mol Ausgangsstoff II, in Frage. Bevorzugte Alkalihypohalogenite sind das Natrium- oder das Kaliumsalz.

Die erfindungsgemäße Umsetzung wird in Gegenwart einer basischen Verbindung, vorteilhaft in einer Menge von 0,2 bis 6, vorzugsweise von 1 bis 4 Äquivalenten basischer Verbindung, bezogen auf ein Mol Ausgangsstoff II, durchgeführt. Bevorzugte basische Verbindungen sind Erdalkaliverbindungen, Ammoniumverbindungen und insbesondere Alkaliverbindungen sowie entsprechende Gemische. Es kommen z. B. als basische Verbindungen in Frage : Kaliumhydroxid, Natriumhydroxid, Kaliumcarbonat, Natriumcarbonat, Lithiumhydroxid, Lithiumcarbonat, Calciumhydroxid, Calciumoxid, Bariumoxid, Bariumhydroxid, Natriummethylat, Natriumäthylat, Natriumpropylat, Natriumisopropylat, Natriumbutylat, Kaliummethylat, Kaliumäthylat, Kalium-n-propylat, Kaliumisopropylat, Kalium-n-butylat. Bevorzugt sind Natron- und Kalilauge.

Die erfindungsgemäße Umsetzung wird im allgemeinen bei einer Temperatur von 15 bis 120 °C, vorzugsweise von 60 bis 110 °C, mit Überdruck oder drucklos, diskontinuierlich oder kontinuierlich durchgeführt. Als Lösungsmittel verwendet man zweckmäßig Wasser. Die Umsetzung kann auch in Gegenwart von unter den Reaktionsbedingungen inerten, organischen Lösungsmitteln erfolgen. Schon aus wirtschaftlichen Gründen wird man die Verwendung von Wasser allein oder Lösungsmittel/Wasser-Gemische in einem Verhältnis von 0 bis 5, zweckmäßig 0 bis 3 Mol Lösungsmittel je Mol Wasser in Betracht ziehen. Man verwendet zweckmäßig Wasser in einem Mengenverhältnis von 90 bis 120 Mol Wasser je Mol Ausgangsstoff II.

Unter Phasentransferkatalysatoren versteht man solche Katalysatoren, die den Transport von Stoffen in binären Systemen, z. B. Wasser und organischem Lösungsmittel, verbessern. Schon kleine Mengen an Katalysator ergeben den Transfereffekt, d. h. die Ausgangsstoffe gelangen mit Hilfe kleiner Katalysatormengen von einer, z. B. der wäßrigen Phase, in eine andere, z. B. einer bestimmten organischen Lösungsmittelphase. Bezüglich der Definition, Herstellung und Eigenschaften solcher Phasentransferkatalysatoren wird auf Anorganische Chemie (1974), Seiten 187 bis 196, auf JACS, Band 93 (1971), Seiten 195 bis 199 und auf Journal of Chemical Education, Vol. 55 (1978), Seiten 429 bis 433 und 350 bis 354, verwiesen. Die Umsetzung wird in Gegenwart von Phasentransferkatalysatoren, vorteilhaft mit einer Menge von 0,001 bis 1, insbesondere von 0,01 bis 0,1 Äquivalenten Katalysator, bezogen auf ein Mol Ausgangsstoff II, durchgeführt.

Als Phasentransferkatalysatoren werden die quarternären Ammoniumsalze III, bevorzugt solche, in deren Formeln die einzelnen reste $R^4$ gleich oder verschieden sein können und jeweils einen Alkylrest mit 1 bis 7 Kohlenstoffatomen bedeuten, verwendet.

Es kommen z. B. als Ausgangsstoffe III in Frage : Tetramethyl-, Tetra-n-propyl-, Tetraisopropyl-, Tetra-n-butyl-, Tetraisobutyl-, Tetra-sek.-butyl-, Tetra-tert.-butyl-, Tetrapentyl-, Tetrahexyl-, Tetra-n-heptyl-, Tetraoctyl-, Tetranonyl-, Tetradecyl-, Tetraundecyl-, Tetradodecylammoniumchlorid ; durch quartäre Substituierung mit vorgenannten Substituenten und/oder mit der Benzyl-, Phenyläthyl-, Phenylpropyl-, Phenylbutyl-gruppe am Stickstoffatom sich entsprechend aus Benzylamin, Tribenzyl-, Tri(phenyläthyl)-, Tri(phenylpropyl)-, Tri(phenylbutyl)-amin ergebende Ammoniumchloride, die auch Ammoniumchloride mit 4 vorgenannten aber völlig oder teilweise unterschiedlichen Resten sein können, z. B. die sich durch Substituierung mit dem Methylrest aus N-Methyl-N,N-diäthylamin, N-Methyl-N-äthyl-N-n-propylamin, ergebenden quartären Ammoniumchloride ; Dimethylbenzyldodecyl-, Cetyltrimethyl-, Methyltriäthyl-, Triäthyldodecyl-, Trimethyltridecyl-, Trimethyl-diphenylmethyl-, Trimethyl-n-dodecyl-, n-Propyl-trimethyl-, Isoamyltrimethyl-, Benzyl-dimethyl-n-octyl-, Benzyltrimethyl-, Benzyltriäthyl-, Trimethyl-(phenyl-(1)-äthyl)-, Trimethyl-(phenyl-(2)-äthyl)-ammoniumchlorid ; entsprechende Ammoniumbromide ; homologe quartäre Ammoniumsalze von anorganischen oder organischen einbasischen oder mehrbasischen Säuren wie Chlorwasserstoff, Bromwasserstoff, Jodwasserstoff, Perchlorsäure, Schwefelsäure, Phosphorsäure, salpetrige Säure, Salpetersäure, Kohlensäure ; Sulfonsäuren wie Benzol- und p-Toluolsulfonsäure ; Bor enthaltende Säuren wie Borsäure, Borfluorwasserstoffsäure ; aliphatische Carbonsäuren wie Chloressigsäure, Dichloressigsäure, Trichloressigsäure ; oder entsprechende Gemische. Bevorzugt sind die Ammoniumsalze der Salzsäure, Schwefelsäure, Phosphorsäure, p-Toluolsulfonsäure, Monochloressigsäure, Di-, Trichloressigsäure.

Die Reaktion kann wie folgt durchgeführt werden : Ein Gemisch von Hypohalogenit, Ausgangsstoff II, quartärem Salz III, basischer Verbindung, Wasser, gegebenenfalls organischem Lösungsmittel, wird während 1 bis 4 Stunden bei der Reaktionstemperatur gehalten. Dann wird aus dem Reaktionsgemisch der Endstoff in üblicher Weise, z. B. durch Phasentrennung und Destillation, Filtration, Extraktion mit einem geeigneten Lösungsmittel und Destillation des Extraktes, abgetrennt.

Gewünschtenfalls kann das gebildete Salz durch entsprechende Mengen Säure in die freie Benzoesäure überführt werden. Als Säuren sind die vorgenannten, dem Ausgangsstoff III in Gestalt ihrer Anionen dienenden Säuren geeignet. Bevorzugt sind Chlorwasserstoff und Schwefelsäure. Die Umsetzung wird zweckmäßig bei einer Temperatur von 0 bis 100 °C, drucklos oder unter Druck, kontinuierlich oder diskontinuierlich, bis zu einem End-pH von 0 bis 3 durchgeführt.

Die nach dem Verfahren der Erfindung herstellbaren Benzoesäuren I sind wertvolle Ausgangsstoffe für Farbstoffe, Schädlingsbekämpfungsmittel und Pharmazeutika, Photoinitiatoren. Beispielsweise ergeben die den Benzoesäuren I entsprechenden Benzoylchloride durch Umsetzuhg mit Alkoxyphosphinen Verbindungen, die als wertvolle Photoinitiatoren verwendet werden.

Bezüglich weiterer Verwendung wird auf die vorgenannten Veröffentlichungen und Ullmanns Encycklopädie der technischen Chemie, Band 4, Seiten 272 bis 291, verwiesen.

Die in den folgenden Beispielen aufgeführten Teile bedeuten Gewichtsteile. Sie verhalten sich zu den Volumenteilen wie Kilogramm zu Liter.

Beispiel 1

a) Ein Gemisch aus 100 Teilen 2,4,6-Trimethylacetophenon, 1 413 Teilen 13-gewichtsprozentiger, wäßriger Natriumhypochlorit-Lösung, 197 Teilen 50-gewichtsprozentiger, wäßriger Natronlauge und 30 Teilen 50-gewichtsprozentiger, wäßriger Dimethyldibenzylammoniumchlorid-Lösung wird eine Stunde bei 25 °C gerührt. Nach dieser Zeit ist der Ausgangsstoff umgesetzt. Die gebildete organische Phase besteht aus 156 Teilen (99 % der Theorie) α,α,α-Trichlor-2,4,6-trimethylacetophenon vom Kp 169 bis 170 °C (21 mbar).

b) Ein Gemisch von 100 Teilen α,α,α-Trichlor-2,4,6-trimethylacetophenon, 1 000 Teilen 6-gewichtsprozentiger, wäßriger Natronlauge und 10 Teilen 50-gewichtsprozentiger, wäßriger Tetrabutylammoniumchlorid-Lösung wird 3 Stunden bei 100 °C gerührt. Man säuert mit 37-gewichtsprozentiger Salzsäure die wäßrige Phase bis pH 1 an. Man erhält 46 Teile (75 % der Theorie) 2,4,6-Trimethylbenzoesäure vom Schmelzpunkt 155 °C.

Beispiel 2 (Vergleich)

Eine Beispiel 1a) analoge Umsetzung ohne Dimethyldibenzylammoniumchlorid ergibt nach 36-stündigem Rühren einen Umsatz von nur 47 Prozent.

Man erhält 6 Teile (10 % der Theorie) 2,4,6-Trimethylbenzoesäure vom Schmelzpunkt 155 °C bei einer Beispiel 1b) analogen Umsetzung ohne Tetrabutylammoniumchlorid.

Beispiel 3

Ein Gemisch aus 100 Teilen 2,4,6-Trimethylacetophenon, 1 060 Teilen 13-gewichtsprozentiger, wäßriger Natriumhypochlorit-Lösung, 148 Teilen 50-gewichtsprozentiger, wäßriger Natronlauge, 30 Teilen 50-gewichtsprozentiger, wäßriger Dimethyldibenzylammoniumchlorid-Lösung wird zunächst eine Stunde bei 25 °C gerührt und anschließend drei Stunden bei 100 °C unter Rückfluß gekocht. Dann wird mit 200 Teilen 37-gewichtsprozentiger Salzsäure das Reaktionsgemisch auf pH 1 gestellt und filtriert. Man erhält 94 Teile (93 % der Theorie) 2,4,6-Trimethylbenzoesäure vom Schmelzpunkt 155 °C.

Beispiel 4

a) Ein Gemisch aus 100 Teilen 2,4,6-Trimethylpropiophenon, 1 300 Teilen 13-gewichtsprozentiger, wäßriger Natriumhypochlorit-Lösung, 182 Teilen 50-gewichtsprozentiger, wäßriger Natronlauge und 14 Teilen 50-gewichtsprozentiger, wäßriger Dimethyldibenzylammoniumchlorid-Lösung wird 3 Stunden bei 40 °C gerührt. Nach dieser Zeit ist das Ausgangsmaterial vollständig umgesetzt. Man destilliert die organische Phase. Man erhält 101 Teile (76 % der Theorie) α,α-Dichlor-2,4,6-trimethylpropiophenon vom Kp 116 °C (1 mbar).

b) Ein Gemisch von 100 Teilen α,α-Dichlor-2,4,6-trimethylpropiophenon, 168 Teilen 50-gewichtsprozentiger, wäßriger Natronlauge, 20 Teilen 50-gewichtsprozentiger, wäßriger Dimethyldibenzylammoniumchlorid-Lösung wird 5 Stunden bei 50 °C gerührt. Man säuert mit 37-gewichtsprozentiger Salzsäure die wäßrige Phase an. Man erhält 40 Teile (60 % der Theorie) 2,4,6-Trimethylbenzoesäure vom Schmelzpunkt 155 °C.

Beispiel 5

Ein Gemisch aus 100 Teilen 2,6-Dichloracetophenon, 160 Teilen 50-gewichtsprozentiger, wäßriger Natronlauge, 20 Teilen 50-gewichtsprozentiger, wäßriger Dimethyldibenzylammoniumchlorid und 1 200 Teilen 13-gewichtsprozentiger, wäßriger Natriumhypochlorit-Lösung werden 2 Stunden bei 25 °C gerührt und anschließend 4 Stunden bei 100 °C unter Rückfluß gekocht. Man säuert mit 37-gewichtsprozentiger Salzsäure die wäßrige Phase auf pH 1 an und filtriert. Man erhält 48 teile (46 % der Theorie) 2,6-Dichlorbenzoesäure vom Schmelzpunkt 141 °C.

**Anspruch**

Verfahren zur Herstellung von o,o-disubstituierten Benzoesäuren der Formel

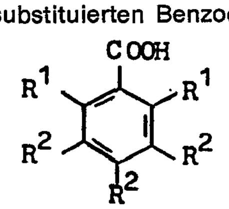

(I)

worin die einzelnen Reste $R^1$ und $R^2$ gleich oder verschieden sein können und jeweils ein Halogenatom, einen aliphatischen, cycloaliphatischen, araliphatischen oder aromatischen Rest oder eine Alkoxygruppe bedeuten, die einzelnen Reste $R^2$ auch jeweils für ein Wasserstoffatom stehen können, oder ihren Salzen, durch Umsetzung von Ketonen mit Hypohalogeniten und basischen Verbindungen und gewünschtenfalls Umsetzung des gebildeten Salzes mit Säure, dadurch gekennzeichnet, daß man o,o-disubstituierte aromatische Ketone der Formel

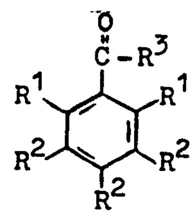

(II)

worin $R^1$ und $R^2$ die vorgenannte Bedeutung besitzen und $R^3$ einen aliphatischen Rest bezeichnet, in Gegenwart von Phasentransferkatalysatoren der Formel

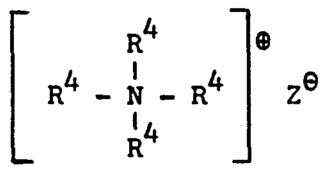

(III)

worin die einzelnen Reste $R^4$ gleich oder verschieden sein können und jeweils einen Alkylrest mit 1 bis 18 Kohlenstoffatomen oder einen Aralkylrest mit 7 bis 12 Kohlenstoffatomen bedeuten, Z für ein Säureanion steht, umsetzt.

**Claim**

A process for the production of o,o-disubstituted benzoic acids of the formula

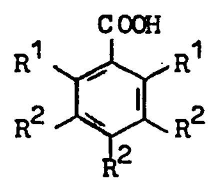

(I)

where the individual radicals $R^1$ and $R^2$ are identical or different and each denotes a halogen atom, an aliphatic, cycloaliphatic, araliphatic or aromatic radical or an alkoxy group, and the individual radicals $R^2$ may also denote a hydrogen atom, or salts thereof by reacting a ketone with a hypohalite and a basic compound and, if desired, reacting the salt formed with acid, characterized in that o,o-disubstituted aromatic ketones of the formula

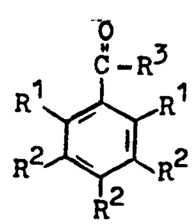

(II)

where $R^1$ and $R^2$ have the meanings given above and $R^3$ denotes an aliphatic radical, are reacted in the presence of a phase transfer catalyst of the formula

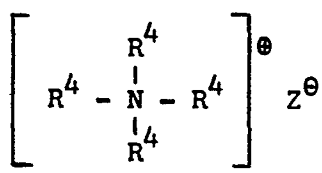

(III)

where the individual radicals $R^4$ are identical or different and each denotes alkyl of from 1 to 18 carbon atoms or aralkyl of from 7 to 12 carbon atoms and Z denotes an acid anion.

**Revendication**

Procédé de préparation d'acides benzoïques o,o-disubstitués de la formule

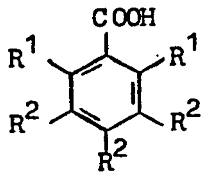

(I)

dans laquelle les divers substituants $R^1$ et $R^2$ peuvent être identiques ou différents et désigner chacun un atome d'halogène ou un groupe aliphatique, cycloaliphatique, araliphatique ou aromatique ou un radical alcoxy, les divers restes $R^2$ pouvant en outre désigner un atome d'hydrogène, et de leurs sels, par réaction de cétones avec des hypohalogénites et des composés basiques et éventuellement du sel formé avec un acide, caractérisé en ce que l'on fait réagir des cétones aromatiques o,o-disubstituées de la formule

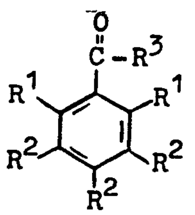

(II)

dans laquelle $R^1$ et $R^2$ possèdent la signification définie et $R^3$ désigne un groupe aliphatique, en présence d'un catalyseur de transfert de phases de la formule

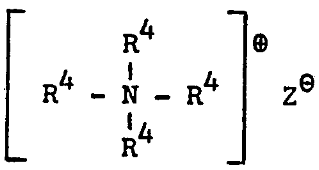

(III)

dans laquelle les divers restes $R^4$ peuvent être identiques ou différents et désigner chacun un radical alcoyle en $C_1$ à $C_{18}$ ou un radical aralcoyle en $C_7$ à $C_{12}$ et Z désigne l'anion d'un acide.